# EUROPEAN PATENT APPLICATION

(11) **EP 2 112 499 A1**
(43) Date of publication of application: **28.10.2009**
(21) Application number: 08003662.7
(22) Date of filing: 28.02.2008
(51) Int. Cl.: G01N 21/47, A61B 3/00, A61B 5/00, G01J 3/45

(54) **Method and apparatus for optical coherence tornography**

(71) Applicant: Optopol Technology S.A., 42-400 Zawiercie (PL)
(72) Inventor: Bajraszewski, Tomasz, 42-400 Zawiercie (PL); Targowski, Piotr, 42-400 Zawiercie (PL); Dalasinski, Pawel, 42-400 Zawiercie (PL); Szkulmowski, Maciej, 42-400 Zawiercie (PL); Wojdas, Pawel, 42-400 Zawiercie (PL)
(74) Representative: Glawe, Delfs, Moll

(57) **Abstract**

The invention relates to optical coherence tomography (OCT), more specifically to spectral optical coherence tomography (SOCT), The invention deals with the problem of artefacts due to sharp increases of reflection by the object under investigation. It solves this problem by splitting of a part off the returning light for modification of the interferometric data.

## Description

The invention relates to optical coherence tomography (OCT), more specifically to spectral optical coherence tomography (SOCT).

OCT is a technique to explore three-dimensional structures of partially reflecting or back-scattering matter with electromagnetic waves in the optical range (wavelengths between 60.0 nm and 1,700 nm). The fundamentals of OCT were presented by Huang et al. in Science, Vol. 254 (1991), p. 1178 - 1181. Huang et al. placed the object to be investigated in the object arm of an interferometer. A piezoelectric transducer was located in the other reference arm in order to modulate the optical path difference (OPD) of the reference beam. Numerical evaluation of the signal generated by the interferometer (interferometric signal) and the modulated OPD facilitated the calculation of the spatial distribution of reflecting or back-scattering structures along the axis of the object beam. Vertical and horizontal scanning of the object beam would allow for three-dimensional imaging of the internal structure of the object.

Application of this time domain OCT to white light interferometry was straightforward (DE 43 09 056 A1). In white light interferometry, the modulation of the OPD in the reference beam is replaced by the use of light at different wavelengths. The interferometric signal is spectrally analyzed to yield a spectral distribution of the light intensity. Numerical evaluation of this spectrum allows for the calculation of the spatial distribution of reflecting or back-scattering structures along the axis of the object beam (cf. DE 43 09 056 A1, US 2008/0013093 A1, Lin An et al., OPTICS LETTERS (Vol. 32) 2007, 3423-3425).

When objects with low reflexion or back-scattering characteristics are measured, the intensity of the reference beam is normally adjusted to a level close to the saturation of the detector (e.g. a CCD camera) in order to obtain shotnoise limited detection. In some applications, however, the intensity of the object beam increases strongly in certain scan positions. For example, when the human eye is examined, the convex curvature of the cornea and its good reflexion characteristics may cause the intensity of the object beam to increase very sharply when the object beam impinges centrally on the pole, the tangent plane of which is perpendicular to the beam. In such situations, the intensity of the interference signal exceeds the level of saturation of the detector. As a consequence, artefacts occur during the numerical evaluation.

The present invention seeks to mitigate this problem of artefacts. The solution is represented by the subject-matter of claims 1 and 8. The further claims describe improvement thereof.

An interferometer within the meaning of the present invention is defined as a device which can split an incoming beam of light into at least two beams of light, the reference beam and the object beam, and superimpose both beams after reflection and/or back-scattering by different media to form an exiting beam of light. If the light is sufficiently coherent, the exiting beam of light contains information about the structures of the media. If one of these structures is known, e.g. if such medium is a mirror, information about the three-dimensional structure of the other medium may be extracted from the exiting beam.

Means for spectral analysis within the meaning of the invention is defined as an assembly which can measure the intensities of light separately for a plurality of, however at least two, spectral ranges. Such ranges shall normally as narrow as possible and consist ideally of a single wavelength only. Means for spectral analysis usually comprise a dispersive element, e.g. a diffraction grating. Means for spectral analysis always comprise at least one sensor element, usually a plurality of sensor elements, e.g. a CCD camera. A sensor element in this context is a device which generates a signal as a function of the intensity of incoming light. Spectral analysis according to the invention is defined as operating such means for spectral analysis.

Measuring the intensity of the split off portion of the light can comprise measurement of the instant intensity or measurement of the light quantity reaching the sensor device during a certain period of time, so that the light intensity is integrated over time. The intensity signal can be dependent on the instant intensity or on the integrated intensity or on a combination thereof.

Interferometric data according to the invention are defined as data representing the light intensities obtained by spectral analysis of the beam of light exiting from an interferometer.

Modifying interferometric data according to' the invention can be performed physically by controlling the quantity of light reaching one ore more of the sensor elements of the means for spectral analysis. Additionally or alternatively, such modification can be performed by modifying the digitized data within the numerical evaluation or both. Controlling the quantity of light has the advantage that saturation of the sensor elements is avoided so that data eligible for computation can still be obtained.

Controlling the quantity of light reaching the sensor element(s) can be performed either by adjusting the intensity of the impinging light, or by adjusting the exposure time, or by a combination of these two measures. Adjusting the light intensity may be made, for instance, with the help of light source current control, a motorized gradient neutral density filter or a liquid crystal tunable filter. As such devices always have a certain response time to be taken into account, adjusting the exposure time is advantageous.

Adjusting the exposure time can easily be carried out by interrupting the exposure during an exposure cycle. An exposure cycle within the meaning of the invention is the period of time in which the sensor elements of the means for spectral analysis are exposed to the incoming light for measurement in one scan position. If a CCD camera is used, an exposure cycle is the period of time during which charge carriers within the CCD pixels are accumulated for the generation of a signal proportional to the quantity of the impinging light. Usually, such exposure cycles alternate with pixel resets.

Exemplary embodiments of the invention is explained in greater detail below with reference to the drawings, in which:
- Fig. 1: is a schematic block diagram showing a first embodiment of an apparatus according to the invention with which the method according to the invention can be performed;
- Fig. 2: shows the time relationship of two triggering signals used for carrying out the invention;
- Fig. 3: is a schematic block diagram showing a second embodiment of an apparatus according to the invention with which the method according to the invention can be performed;
- Fig. 4: is a schematic block diagram showing a third embodiment of an apparatus according to the invention with which the method according to the invention can be performed;
- Fig. 5: is a schematic block diagram showing a fourth embodiment of an apparatus according to the invention with which the method according to the invention can be performed.

A beam of light is generated by a light source 1, coupled into an optical fiber system 2 and then passed through an optical insulator 3 preventing returning light from entering and destroying the light source 1. The light beam exiting from the optical insulator 3 reaches a first coupler 4 acting as a beam splitter having a splitting ratio of 10/90. 90 percent of the light propagates to a second coupler 5 acting as a beam splitter having a splitting ratio 50/50. The second coupler 5 divides the light beam into an object beam 6 and a reference beam 7. Polarisation controllers (not shown) may be placed along the fiber system for both the reference beam 7 and the object beam 6. After adjustment by lenses 8 and 9 and reflection by a reference mirror 10, the reference beam 7 returns to the second coupler 5. Means for enhancing the accuracy of the system may be placed between the lenses 8 and 9, e.g. neutral density filters and dispersion compensators (not shown). The object, beam 6 is parallelized by a lens 11 and then directed to a galvanometric mirror 12 to facilitate scanning along the scan positions. A further lens 13 focuses the object beam 6 to the area under investigation within the object 14. From here, the object beam 6 is reflected and/or back-scattered so that it returns to the second coupler 5, where the object beam 6 is superposed with the reference beam 7 to jointly exit from the interferometer.

The light exiting from the interferometer through the second coupler 5 passes in equal portions through ports 5a and 5b of said second coupler 5. Port 5a is connected to means for spectral analysis. This assembly comprises a lens 15 for parallelization of the light after exiting from the optical fiber system 2. Said assembly further comprises a diffraction grating 16 being illuminated with the parallelized light beam. It further comprises a lens 17 for focusing the diffracted light on an array of sensor elements 18 to generate the interferometric data.

The interferometric data are fed into a computing device 19. In addition, a driving unit 20 for adjusting the scanning angle of the galvanometric mirror 12 exchanges data about the current mirror orientation with the computing device 19. While or after the scanning is performed, the computing device 19 can compute the three-dimensional structure of the object 14 on the basis of the interferometric data and the mirror orientation and visualize said structure on a display screen 21. If the object 14 is human or animal tissue, the operator can make a diagnosis on the basis of the structure on the display screen 21 and, if available, other facts known about the patient.

Port 5b of said second coupler 5 is connected to the first coupler 4 to which thus 50 percent of the light exiting the interferometer returns. Due to the splitting ratio of 10/90 of this first coupler 4, a portion of 10 percent of the returning light is branched off. This portion travels to a first sensor device 22, which is a photodetector with a response time which is substantially shorter than the length of the exposure cycles of the sensor elements 18. For example, a photodiode with a response time below 10 nano seconds may be employed so the response time is far below the exposure time of CCD devices. This first sensor device 22 generates an intensity signal fed to an exposure control unit 23. This intensity signal is dependent on, e.g. proportional to, the light intensity measured by the first sensor device 22. Correspondingly, it depends on the light intensity reaching the sensor elements of said array 18.

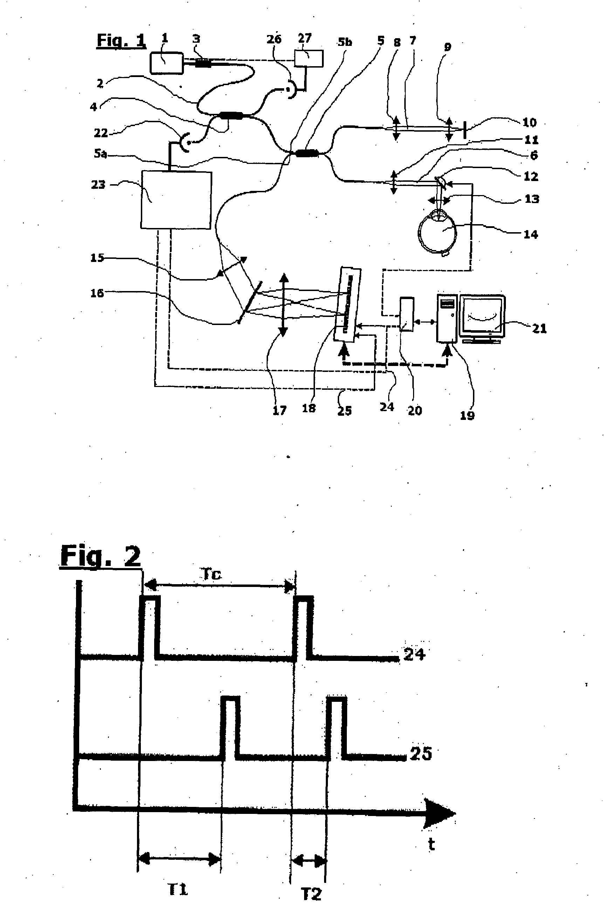

In the exemplary embodiment shown, the exposure control unit 23 and the driving unit 20 jointly generate a first trigger signal triggering the start of an exposure cycle off the array of sensor elements 18, said first trigger signal being symbolized by broken line 24. Said exposure control unit 23 further generates a second trigger signal triggering the end of the exposure cycle, this second trigger signal being symbolized by dotted line 25. As long as said intensity signal generated by said first sensor device 22 remains below a predetermined value, the second trigger signal is generated after constant periods of exposure time after the first trigger signal so there is no interruption of the exposure cycles. If however, said intensity signal exceeds said predetermined value, the period of exposure time is shortened, i.e. the second trigger signal is generated earlier. Fig. 2 illustrates this way of operation. The first trigger signal, represented by graph 24, is generated in a repetitive manner after constant periods of time Tc. As long as the light intensity and thus said intensity signal does not exceed a predetermined value, the second trigger signal, represented by graph 25, is generated after a constant period of exposure time T1. Otherwise it is generated after a shortened period of exposure time T2. The higher the degree in which said predetermined value is exceeded, the earlier the second trigger signal will be generated. It will be well understood by the skilled person that there are additional or alternative modes of how to modify the interferometric data depending on the intensity signal obtained with sensor device 22. In particular, the intensity signal from sensor device 22 may be fed directly to the computing device 19 so that the occurrence of an early exposure cycle interruption may be accounted for during the computation.

Placing said first coupler 4 between the light source 1 and the second coupler 5 is advantageous over placing it between the second coupler 5 and the means for spectral analysis, because it does not entail a reduction of the intensity of the light from which the interferometric data are extracted.

The remaining port of said first coupler 4, through which port 10 percent of the light coming from the light source 1 exits, is connected to a second sensor device 26. This second sensor device 26 generates a voltage signal depending on the intensity of the light coming from the light source 1. This voltage signal is fed to a light control unit 27. Said light control unit 27 generates a control signal fed to the light source 1 causing the light intensity to drop if a predetermined value is exceeded. It is thus ensured that the interferometer, the means for spectral analysis and, in particular, the object 14 are not exposed to harmful intensities of light. Furthermore, the first coupler 4 serves two functions in an elegant manner.

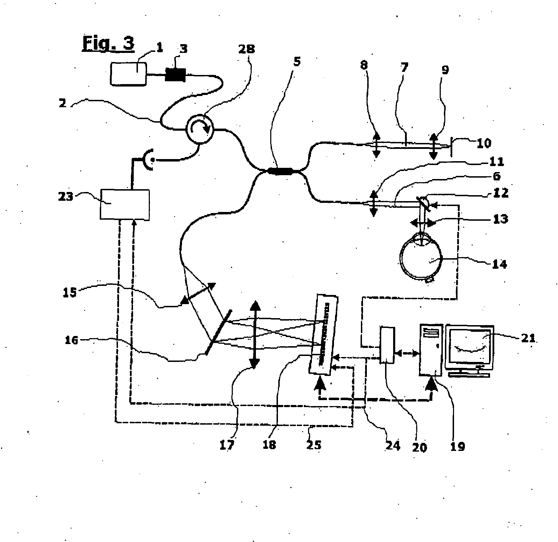

It will be well understood that variations of the exemplified setup are possible without leaving the scope of the invention. For instance, the first coupler 4 may be replaced by an optical circulator 28 as shown in Fig. 3. In this case, the light from the light source 1 is completely passed on to the second coupler 5 of the interferometer, and all of the light returning from the second coupler 5 is passed on to the first sensor device 22.

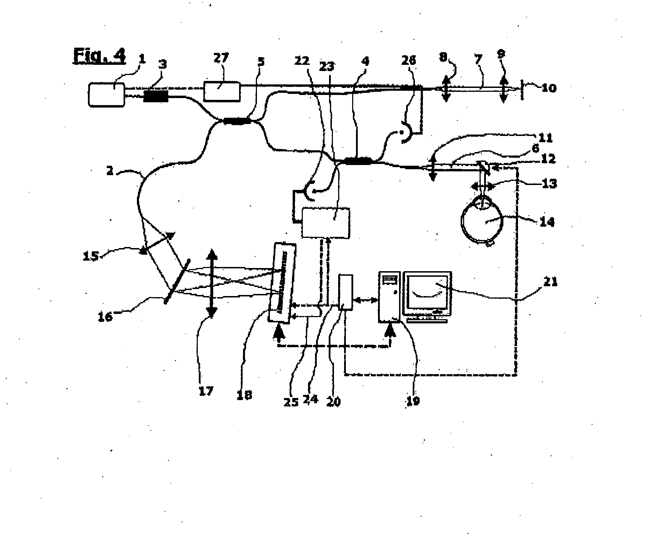

Alternatively, as shown in Fig. 4, the first coupler 4 may be placed in the object arm of the interferometer between the second coupler 5 and the lens 11. This configuration has the great advantage that the light returning from the object 14 is measured directly without being influenced by the light from the reference beam. The splitting ratio of the first coupler 4 is preferably 10/90 or below, more preferably 1/99, so that there is hardly any attenuation of the object beam. The remaining fourth port of the first coupler 4 may be used in combination with a second sensor device 26 to avoid harmful light intensities as described above.

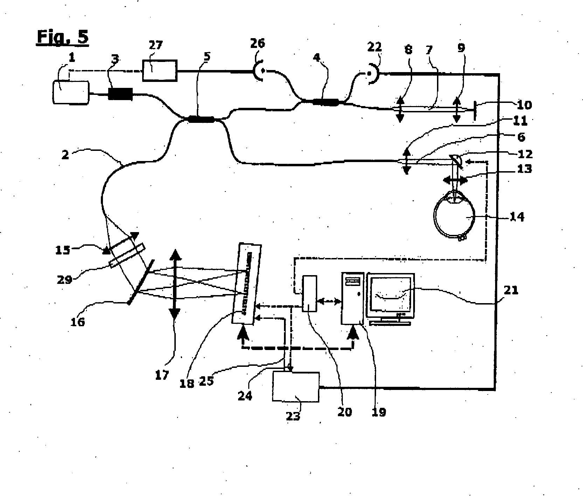

As shown in Fig. 5, a device for splitting off a portion of the light returning from the object arm may also be implemented with a glass plate 29 inserted between lens 15 and diffraction grating 16 in combination with a first coupler 4 being placed in the reference arm. The glass plate 29 reflects a portion, e.g. 4 percent, of the light propagating towards the grating 16 back to the second coupler 5 from where it travels on to the reference arm where the first coupler 4 splits off the portion and which is then directed to the first sensor device 22. Here again, the fourth port of the first coupler 4 may be used in combination with a second sensor device 26 to avoid harmful light intensities as described above.

## Claims

1. Method for removing artefacts from interferometric data in spectral optical coherence tomography (SOCT) performed on an object using an interferometer and means for spectral analysis comprising:
a) splitting the light returning from the object into at least a first and a second portion,
b) using the first portion for spectral analysis,
c) measuring the intensity of the second portion and generating an intensity signal depending on the intensity of the second portion,
d) modifying the interferometric data in dependence of the intensity signal generated according to c).

2. Method of claim 1 wherein modifying the interferometric data according to d) comprises controlling the quantity of light reaching one or more of the sensor elements (18) of the means for spectral analysis.

3. Method of claim 2 wherein controlling the quantity of light reaching the means for spectral analysis comprises adjusting the intensity of the light impinging on one or more of the sensor elements (18) of the means for spectral analysis.

4. Method of claim 2 or 3 wherein controlling the quantity of light reaching the means for spectral analysis comprises adjusting the exposure time for at least one of the sensor elements (18) of the means for spectral analysis.

5. Method of claim 4 wherein adjusting the exposure time comprises interrupting the exposure of at least one of the sensor elements (18) of the means for spectral analysis during an exposure cycle.

6. Method of claim 5 wherein the exposure is interrupted in that moment in which the light intensity measured according to c) exceeds a predetermined value.

7. Method of claim 5 wherein the exposure is interrupted after a period of time the duration of which is calculated in dependence of the intensity signal generated according to c).

8. Apparatus for optical coherence tomography comprising a light source (1), an interferometer with an object arm, means for spectral analysis, means (19) for numerical evaluation of the interferometric data obtained from the means for spectral analysis, **characterized in that** said apparatus further comprises means (4, 29) for splitting off a portion of the light returning from the object arm and propagating to the means for spectral analysis, a first sensor device (22) arranged for generating an intensity signal as a function of the light intensity of said split off portion of light, means (23) for modifying said interferometric data according to the signal generated by said first sensor device (22).

9. Apparatus according to claim 8 further comprising exposure limiting means (23) arranged for adjusting the quantity of the light impinging on at least one sensor element (18) of the means for spectral analysis.

10. Apparatus according to claim 9 wherein said exposure limiting means (23) is arranged for interrupting the exposure for the sensor element (18) during an exposure cycle.

11. Apparatus according to claim 10 wherein said exposure limiting means (23) is arranged for interrupting the exposure in dependence of the intensity signal.

12. Apparatus according to any of claims 8 to 11 wherein said means (4, 29) for splitting off a portion of the light is an optical coupler (4).

13. Apparatus according to claim 12 wherein said coupler (4) is arranged for directing a portion of the light exiting from the light source (1) to a second sensor device (26) arranged for generating a signal as a function of the light intensity of said split off portion of light and light control means (27) for modifying the intensity of the light exiting from the light source (1) according to the signal generated by said second sensor device (26).

14. Apparatus according to claim 13 wherein said light control means (27) is arranged for reducing the intensity of the light exiting from the light source (1) if this intensity exceeds a predetermined value.

## Amended claims

### Amended claims in accordance with Rule 137(2) EPC.

**1.** Method for removing artefacts from interferometric data in spectral optical coherence tomography performed on an object using an interferometer and means for spectral analysis comprising:
a) splitting the light returning from the object into at least a first and a second portion,
b) using the first portion for spectral analysis,
c) measuring the intensity of the second portion and generating an intensity signal depending on the intensity of the second portion,
d) modifying the interferometric data in dependence of the intensity signal generated according to c).

**2.** Method of claim 1 wherein modifying the interferometric data according to d) comprises controlling the quantity of light reaching one or more of the sensor elements (18) of the means for spectral analysis.

**3.** Method of claim 2 wherein controlling the quantity of light reaching the means for spectral analysis comprises adjusting the intensity of the light impinging on one or more of the sensor elements (18) of the means for spectral analysis.

**4.** Method of claim 2 or 3 wherein controlling the quantity of light reaching the means for spectral analysis comprises adjusting the exposure time for at least one of the sensor elements (18) of the means for spectral analysis.

**5.** Method of claim 4 wherein adjusting the exposure time comprises interrupting the exposure of at least one of the sensor elements (18) of the means for spectral analysis during an exposure cycle.

**6.** Method of claim 5 wherein the exposure is interrupted in that moment in which the light intensity measured according to c) exceeds a predetermined value.

**7.** Method of claim 5 wherein the exposure is interrupted after a period of time the duration of which is calculated in dependence of the intensity signal generated according to c).

**8.** Apparatus for spectral optical coherence tomography comprising a light source (1), an interferometer with an object arm, means for spectral analysis, means (19) for numerical evaluation of the interferometric data obtained from the means for spectral analysis, **characterized in that** said apparatus further comprises means (4, 29) for splitting off a portion of the light returning from the object arm and propagating to the means for spectral analysis, a first sensor device (22) arranged for generating an intensity signal as a function of the light intensity of said split off portion of light, means (23) for modifying said interferometric data according to the signal generated by said first sensor device (22).

**9.** Apparatus according to claim 8 further comprising exposure limiting means (23) arranged for adjusting the quantity of the light impinging on at least one sensor element (18) of the means for spectral analysis.

**10.** Apparatus according to claim 9 wherein said exposure limiting means (23) is arranged for interrupting the exposure for the sensor element (18) during an exposure cycle.

**11.** Apparatus according to claim 10 wherein said exposure limiting means (23) is arranged for interrupting the exposure in dependence of the intensity signal.

**12.** Apparatus according to any of claims 8 to 11 wherein said means (4, 29) for splitting off a portion of the light is an optical coupler (4).

**13.** Apparatus according to claim 12 wherein said coupler (4) is arranged for directing a portion of the light exiting from the light source (1) to a second sensor device (26) arranged for generating a signal as a function of the light intensity of said split off portion of light and light control means (27) for modifying the intensity of the light exiting from the light source (1) according to the signal generated by said second sensor device (26).

**14.** Apparatus according to claim 13 wherein said light control means (27) is arranged for reducing the intensity of the light exiting from the light source (1) if this intensity exceeds a predetermined value.
